# EUROPEAN PATENT APPLICATION

(11) **EP 3 939 608 A1**
(43) Date of publication of application: **19.01.2022**
(21) Application number: 19920108.8
(22) Date of filing: 02.05.2019
(51) Int. Cl.: A61K 39/39, A61K 39/135, A61K 39/00

(54) **IMMUNITY ENHANCING ADJUVANT THAT CAN BE ADMINISTERED IN COMBINATION WITH OIL EMULSION, AND VACCINE COMPOSITION FOR FOOT-AND-MOUTH DISEASE COMPRISING SAME**

(30) Priority: 15.03.2019 KR 20190030149
(71) Applicant: REPUBLIC OF KOREA (ANIMAL AND PLANT QUARANTINE AGENCY), Gimcheon-si, Gyeongsangbuk-do 39660 (KR)
(72) Inventor: LEE, Minja, Yangsan-si Gyeongsangnam-do 50535 (KR); PARK, Jong-Hyeon, Gimcheon-si Gyeongsangbuk-do 39660 (KR); KIM, Su-Mi, Daejeon 34899 (KR); JO, Hyundong, Gimcheon-si Gyeongsangbuk-do 39660 (KR); KIM, Byounghan, Seoul 06760 (KR)
(74) Representative: Richly & Ritschel Patentanwälte PartG mbB
(86) International application number: PCT/KR2019/005243
(87) International publication number: WO 2020/189840

(57) **Abstract**

The present invention relates to an adjuvant composition for a foot-and-mouth disease vaccine. The present invention can provide induction of a strong immune response through simultaneous induction of cellular and humoral immunity in cloven-hoofed animals such as cattle, pigs, etc., for prevention and treatment of a foot-and-mouth disease outbreak, a safe and optimized adjuvant composition, and a vaccine composition comprising same. In addition, when the foot-and-mouth disease breaks out, the present invention can be used to effectively cope with the outbreak as an emergency vaccine in the field, and can provide a stabilized systematic vaccine to cloven-hoofed animals.

## Description

### [Technical Field]

The present invention relates to an immune-enhancing adjuvant for a foot-and-mouth disease vaccine and a vaccine composition including the same. More particularly, the present invention relates to an adjuvant for enhancing immunity of a foot-and-mouth disease vaccine, which can be administered in combination with an oil emulsion, and a vaccine composition including the same.

### [Background Art]

Foot-and-mouth disease (FMD) is a highly contagious viral disease that primarily affects cloven hoofed livestock (that is, Artiodactyla), resulting in significant economic losses for the livestock industry due to its rapid spread, high mortality and decreased productivity. The susceptible species include about 70 species or more of wildlife, including ruminants (livestock ruminants) such as cattle, pigs, water buffalo, camels, sheep, and goats. This disease is accompanied by high fever and is known to cause blistering in the mouth, tongue, snout, nose, nipples, hooves and other hairless areas of the skin.

Vaccination is used as a means to control pathologic conditions in countries suffering from foot-and-mouth disease, and plays an important role in establishing contingency plans in countries without foot-and-mouth disease. Foot-and-mouth disease vaccines worldwide use dead poison (inactivated) vaccines, and most thereof have improved in terms of effectiveness by vaccination with oil adjuvants (Double Oil Emulsion, DOE or Single Oil Emulsion, SOE). However, late antibody induction period to the defensive levels, low antibody titer, short persistence of antibodies and low immunogenicity in pigs compared to cattle are pointed out as disadvantages.

Foot-and-mouth disease vaccines till now have focused on induction of humoral immune responses rather than cellular immune responses, but protective performance thereof is not perfect. The induction period of humoral immune response (main neutralizing antibody IgG) by foot-and-mouth disease vaccine takes 4 to 7 days, whereas cellular immune response is induced within several hours to 3 days. Therefore, at the early stage of foot-and-mouth disease infection, the induction of cellular immune response seems to be more effective in defending the host than the humoral immune response. Further, the current foot-and-mouth disease vaccine requires regular vaccination every 4 to 6 months since an antibody maintenance period is short after vaccination. In particular, when inoculating pigs intramuscularly, there are disadvantages of low safety and local side effects such as formation of lesions at the vaccination site such as fibrosis and granuloma at the inoculation site. On the other hand, research related to the foot-and-mouth disease vaccines till now has been conducted in a large number of assessment studies to determine vaccine efficacy in cattle than in pigs among livestock species, however, immunogenicity through vaccination is reported to be lower in pigs than in cattle. Therefore, an ideal vaccine design to overcome the limitations of commercial vaccines should satisfy requirements including, for example, simultaneous induction of cellular and humoral immunity, maintaining high antibody titer by inducing memory responses, securing safety in order to reduce local side effects, and development of adjuvant optimized for livestock species according to new strategies and the like.

In foreign countries, Marcol 52, ISA 206 and ISA 50 are mostly used as the adjuvant in commercialized foot-and-mouth disease vaccines. As adjuvant-related research for effective defense of foot-and-mouth disease, a variety of studies such as assessment of foot-and-mouth disease vaccine efficacy in pigs and goats using, for example: pattern recognition receptors (PRRs) ligands such as R848, Poly(I:C), MDP, MPL, β-glucan, etc.; immunity enhancers such as rapeseed oil and ginseng root saponin, etc.; or typical commercial adjuvants (ISA 201, ISA 206, Emulsigen-D, Carbigen, etc.), has been studied , however, these have not yet induced complete immunity.

Other than the foot-and-mouth disease vaccine, there are also efforts to increase immunogenicity by simultaneous inducing cellular and humoral immunity with primarily human vaccines, specifically: 1) vaccine delivery systems such as oil emulsion, surfactant, liposome, virosome, ISCOMs, etc.; 2) immunity enhancers such as saponin, alumium hydroxide, potassium phosphate, etc.; 3) toll-like receptors (TLRs), RIG-I-like receptors (RLRs), nucleotide-binding oligomerization domain (NOD)-like receptors (NLRs) and receptor-specific immunostimulating agents such as C-type lectin receptors (CLRs) ligand; 4) various cytokines such as IL-1, IL-2, IL-6, IL-18, TNFα, IFNα, IFNγ and GM-CSF, and the like have been studied and used as adjuvants. Some of these materials are currently being used as vaccine adjuvants for prevention and treatment of various human diseases such as cancer, tuberculosis, hepatitis B, malaria, influenza, HIV and HSV, or in clinical trials, but are not employed as a vaccine composition for a foot-and-mouth disease. Further, since various adjuvants have different modes of action, it is very important to understand the immunological mechanisms of the adjuvants for FMD vaccine development according to new strategies that can induce strong cellular and humoral immune responses.

Therefore, the present invention intended to suggest the possibility of FMD vaccine development according to new strategies through development of various PPRRs ligands and cytokines having adjuvant efficacy in mice, induction of memory immune responses through induction of cellular and humoral immune responses, and optimized adjuvants for different species (Artiodactyla such as cattle and pigs) and a vaccine composition for a foot-and-mouth disease including the same.

Meanwhile, prior art related to the above technologies includes Korean Patent Laid-Open Publication No. 10-2017-0097116 entitled "Foot-and-mouth disease vaccine", Korean Patent Laid-Open Publication No. 10-2018-0024030 entitled "Oily adjuvant", and the like, but effects of the above materials as immune-enhancing assistants or adjuvants useable in foot-and-mouth disease vaccines and a vaccine composition including the same are not yet specifically described.

### [Prior Art Document]

### [Patent Document]

(Patent Document 1) Korean Patent Laid-Open Publication No. 10-2017-0097116
(Patent Document 2) Korean Patent Laid-Open Publication No. 10-2018-0024030

### [Summary of Invention]

### [Problems to be Solved by Invention]

In order to address the aforementioned problems, the present invention aims to provide an adjuvant which is safe and optimized for protective antibody induction in Artiodactyla (i.e., cloven hoofed livestock) such as cattle and pigs as well as mice in order to prevent and treat foot-and-mouth disease, and a vaccine composition including the same.

### [Means for Solving Problems]

In order to achieve the above object, the present invention provides an adjuvant composition that includes a pattern recognition receptors (PRRs) ligand as an active ingredient.

Further, the present invention also provides a vaccine composition for a foot-and-mouth disease including the above composition.

The adjuvant composition may contain cytokines as an active ingredient, along with the pattern recognition receptors (PRRs) ligand.

In the present invention, Artiodactyla generally refers to a group of animals with two hoofs such as cattle, pigs, sheep, goats, reindeer, water buffalo and camels, and is preferably cattle and pigs.

Foot-and-mouth disease virus antigens in the present invention may include foot-and-mouth disease virus O serotype, foot-and-mouth virus A serotype, foot-and-mouth disease virus Asian 1 serotype, foot-and-mouth disease virus C serotype, foot-and-mouth disease virus SAT1 serotype, foot-and-mouth disease virus SAT2 serotype and/or foot-and-mouth disease virus SAT3 and the like.

Cytokine is a generic term for biologically active factors that are secreted from cells and are involved in intercellular signaling, cell behavior regulation, immune response regulation, etc., and may refer to low molecular proteins such as interleukin, lymphokine, interferon, cell proliferation and differentiation factors, etc.

The cytokines of the present invention may include IL-1 beta, IL-2, IL-4, IL-5, IL-6, IL-9, IL-10, IL-12p70, IL-12/23p40, IL-15/15R, IL-17A, IL-21, IL-22, IL-23 (IL-12p40/IL-23p19), IL-33, TNF-alpha, IFN-alpha, IFN-beta, IFN-gamma, etc., but they are not limited thereto.

The above cytokine means commonly useable ones, and may include recombinant cytokines expressed in *E. Coli,* Baculovirus, HEK 293 cells and the like.

In the pattern recognition receptors (PRRs) ligand of the present invention, the pattern recognition receptor may serve to recognize conserved molecular patterns that distinguish viruses, bacteria, fungi and parasites, and the ligand refers to an agonist of the receptor, that is, a substance that is bound to a receptor and activates the same.

The PRRs ligand may include, for example, R848 (TLR-7/8 agonist), TDB (Mincle agonist), Curdlan (Dectin-1 agonist), Furfurman (Dectin-2 agonist), MDP (NOD-2 ligand), MPLA-SM (TLR-4), Zymosan (Dectin-2/TLR-2), Chitosan (NLRP inflammasome inducer, MR), Poly(I:C) (TLR-3), Poly(dA:dT) (RIG-1/CDS agonist, AIM-2 inflammasome inducer) or STING-based ligand, but it is not limited thereto.

The STING-based ligand may include DNA, RNA, protein, peptide fragments, compounds, etc. that can activate STING signaling, and preferably includes c-di-GMP (cyclic diguanylate), cGAMP, 3'3'-cGAMP, c-di-GAMP, c-di-AMP, 2'3'-cGAMP, 10-(carboxymethyl)9(10H)acridone (CMA)(10-(carboxymethyl)9(10H)acridone(CMA)), 5,6-dimethylxanthenone-4-acetic acid-DMXAA, methoxyvone, 6,4'-dimethoxyflavone, 4'-methoxyflavone, 3',6'-dihydroxyflavone, 7,2'-dihydroxyflavone, daidzein, formononetin, retusin 7-methyl ether, xanthone, and the like, but it is not limited thereto.

The poly (I:C) refers to not only naturally occurring polymers of polyinosinic acid:polycytidylic acid, but also synthetic forms thereof.

The composition of the present invention may include one or more components optionally selected from the above PRRs.

When two components among the above PRRs ligands are included, any one PRRs ligand : the remaining PRRs ligand may be included in a weight ratio of 1 : 1 to 3, and preferably 1 : 1 to 2.

With regard to administered amount, that is, a dosage or dose in the present invention, the PRRs ligand or cytokine is not limited, but is preferably included in the dose of 1 to 100 µg/ml

When two or more of the PRRs ligands are included, each ligand may be included in the range of 1/2 or less based on the dose contained alone, preferably 1 to 30 µg/ml, more preferably 5 to 15 µg/ml, and most preferably 7 to 10 µg/ml.

Since the above dose is within a range that is not toxic *in vivo,* if it is out of the above range, there is a risk of toxicity in the body when administered to an animal.

Other than the above components, oils (or oil emulsions) and emulsifiers, gels, etc. well known in the art may be further included.

The oil (or oil emulsion) may be ISA 201, ISA 61, ISA 50 or ISA 206, but it is not limited thereto.

The emulsifiers may include substances generally recognized as emulsifiers, such as other products of the TWEEN^{®} or SPAN^{®} product line (fatty acid esters of polyethoxylated sorbitol and fatty acid-substituted sorbitan surfactants, respectively), and other solubility improving agents such as PEG-40 castor oil or other PEGylated hydrogenated oil, but they are not limited thereto.

### [Advantageous Effects]

The present invention may provide an adjuvant composition which is safe and optimized for induction of protective antibodies of Artiodactyla such as cattle, pigs, etc. in order to prevent and treat Picornavirus including foot-and-mouth disease, and a vaccine composition including the same. Further, when foot-and-mouth disease occurs, the present invention may be used to effectively cope with emergency vaccines in the field, and effective and stabilized vaccines may be provided as regular vaccines for Artiodactyla.

### [Brief Description of Drawings]

FIG. 1 illustrates results of inducing an initial cellular immune response *in vivo* according to serotype-specific antigens.
FIG. 2A illustrates results of inducing innate immune response and cellular immune response of DCs according to serotype-specific antigens.
FIG. 2B illustrates results of inducing innate immune response and cellular immune response of MΦs according to serotype-specific antigens.
FIG. 3 illustrates a cytokine expression profile induced by foot-and-mouth disease virus (O TWN 97-R) antigen in mice and a strategy for time kinetics studies *in vivo.*
FIG. 4 illustrates the cytokine expression profile and *in vivo* time kinetics results induced by foot-and-mouth disease virus (O TWN 97-R) antigen in mice (Time point; 0, 6, 12, 24, 48, 72, 96h) .
FIG. 5 illustrates a strategy for identification of *in vitro* characteristics of cytokine expression mediated by foot-and-mouth disease virus (O TWN 97-R) antigen in DCs, MΦs, iNK T cells, γδ T cells, and T cells isolated from mouse peritoneal exudate cells (PECs) and splenocytes.
FIGS. 6A to 6C illustrate results of time-related cytokine expression mediated by foot-and-mouth disease virus (O TWN 97-R) antigen in DCs, MΦs, iNK T cells, γδ T cells and T cells isolated from mouse peritoneal exudate cells (PECs) and splenocytes (Time point; 0, 6, 12, 24, 48, 72, 96h) .
FIG. 7 illustrates a strategy for identification of *in vitro* characteristics of cytokine expression mediated by foot-and-mouth disease virus (O TWN 97-R) antigen when co-culturing DCs, iNK T cells, γδ T cells and T cells isolated from mouse peritoneal exudate cells (PECs) and splenocytes, wherein (A) shows a strategy for *in vitro* study, and (B) is a schematic diagram for co-cultured cells.
FIG. 8 illustrates results of antigen-mediated cytokine expression in co-cultured cells, DCs, iNK T cells, γδ T cells and T cells in mice.
FIG. 9 illustrates a strategy for confirming antigen O TWN 97-R-mediated *in vivo* defense effects at the early stage of FMDV infection.
FIG. 10 illustrates influence on the antigen O TWN 97-R-mediated *in vivo* defense effects (mouse survival rate, change of body weight) at the early stage of FMDV infection.
FIG. 11 illustrates a strategy for cell depletion, cytokine neutralization, and antigen-mediated *in vivo* defense effects.
FIGS. 12A and 12B illustrate *in vivo* defense effects (survival rate, change of body weight) by antigen upon depletion of DCs and MΦs and neutralization of cytokines on host protection against FMDV infection.
FIG. 13 illustrates results of expression of antigen-mediated stimulatory molecules CD40, CD80 and CD86 and cell proliferation in DCs treated with O TWN 97-R antigen and stimulants (R848, Chitosan, Zymosan, ODN 2395, TDB, Furfuman and c-di-GMP).
FIG. 14 illustrates activity of IL-23 through simultaneous activation of Dectin-1 and Mincle in DCs and MΦs.
FIG. 15 illustrates a strategy for evaluating potential as a vaccine adjuvant and short-term immune memory effects on FMDV infection when PRRs ligand and cytokine are administrated alone or in combination.
FIG. 16 illustrates short-term immune memory effects and *in vivo* defense effects (survival rate, change of body weight) when PRRs ligand and cytokines are administrated alone or in combination as an adjuvant for host protection against FMDV infection.
FIG. 17 illustrates a strategy for evaluating long-term memory response mediated by cytokines, HSP70 protein and PRRs ligand against FMDV infection (long-term immunity) and protective effects thereof in mice.
FIG. 18A to 18D illustrate results of long-term memory immune responses mediated by cytokines, HSP70 protein and PRRs ligand, and protective effects on FMDV infection in mice.
FIG. 19A to 19G illustrate results of expansion of cytokine, HSP70 protein and PRRs ligand-mediated immune cells in mice.
FIG. 20 illustrates a strategy for confirming IFNα and IL-23-mediated protective effects on infection with various FMDV serotypes (types O, A, and Asia1).
FIG. 21 illustrates results of IFNα and IL-23-mediated memory responses and a wide range of protective effects on infection with various FMDV serotypes (types O, A, and Asia1).
FIG. 22 illustrates LDH release (cytotoxicity, after 96 hours) in PRRs ligand-treated bovine PBMCs.
FIG. 23 illustrates results of cell proliferation mediated by PRRs ligand (after 96 hours) in bovine PBMCs.
FIG. 24 illustrates a strategy for evaluating PRRs ligand-mediated adjuvant effects and memory responses in cattle.
FIG. 25 illustrates PRRs ligand-mediated adjuvant effects and memory response results in cattle.
FIG. 26 illustrates results of LDH release (cytotoxicity, after 96 hours) in porcine PBMCs treated with PRRs ligand.
FIG. 27 illustrates results of PRRs ligand-mediated cell proliferation (after 96 hours) in porcine PBMCs.
FIG. 28 illustrates a strategy for evaluating the PRRs ligand-mediated adjuvant effects and memory response in pigs.
FIG. 29 illustrates results of the PRRs ligand-mediated adjuvant effects and memory response in pigs.

### [Mode for Carrying out Invention]

Hereinafter, the present invention will be described in detail by ways of examples and experimental examples.

However, the following examples and experimental examples are merely illustrative of the present invention, and the contents of the present invention are not limited to the following examples and experimental examples.

Experimental materials commonly used in the examples and experimental examples of the present invention are as follows;

### <Antigen>

Antigens were purified and inactivated according to the following method.

The antigen was prepared from FMDV O TWN 97-R (GenBank AY593823 for P1) and BHK-21 cells. For virus infection, the culture medium was replaced with serum-free Dulbecco Modified Eagle medium (DMEM, Cellgro, Manassas, VA, USA) and incubated in 5% CO₂ gas at 37 °C for 1 hour in order to inoculate the virus. Extracellular virus was removed. After 24 hours from infection, the virus was inactivated by treatment with 0.003N binary ethylenimine twice for 24 hours in a shaking incubator and concentrated with polyethylene glycol (PEG) 6000 (Sigma-Aldrich, St. Louis, MO, USA). The virus concentrate was layered by a 15-45% sucrose density gradient, followed by centrifugation. After ultracentrifugation, the bottom of the centrifuge tube was pierced, and a 1 mL fraction was collected. The presence of FMDV particles in each fraction sample was confirmed by optical density using a lateral flow device (BioSign FMDV Ag, Princeton BioMeditech, Princeton, NJ, USA). Before use in the experiment, the pre-PEG-treated supernatant was passed through ZZ-R and BHK-21 cells at least twice in order to confirm whether no cytopathic effect (CPE) occurred, and therefore, it was confirmed that there was no viable virus in the supernatant.

### <Pattern recognition receptors (PRRs) ligand and cytokines>

As the PRRs ligand, Invivogen (SanDiego, CA, USA) was used while a product of MitenyiBiotec (MiltenyiBiotec, BergischGladbach, Germany) was used as the cytokine. The oil emulsion ISA 206 was purchased from Seppic Corporation (Seppic Inc., Paris, France), while aluminium hydroxide gel (Alhydrogel ^{®}) and Quil-A were purchased from Invivogen.

### <Mouse>

Wild-type C57BL/6 mice (7 weeks old female) with matching age and sex were purchased (KOSA BIO Inc, Gyeonggido, Korea). All mice were housed in micro-quarantine cages in a specific sterile pathogen (SPF) animal facility with Biosafety Level 3 (ABSL3) of the Korean Agriculture, Forestry and Livestock Quarantine Division.

### <Preliminary Experimental Example> Test to confirm host immunological mechanism of foot-and-mouth disease antigen

### 1. Confirmation of induction of intrinsic immune response according to serotype-specific antigens

1-1. In order to confirm whether 8 antigens induce intrinsic immune response *in vivo,* 2 µg of each antigen was injected into the intraperitoneal cavity of the mouse, and the intraperitoneal cavity was washed with 2 mL of cooled PBS, and the collected intraperitoneal washing solution was centrifuged, followed by analyzing the cytokine expression patterns and time kinetics in the supernatant.

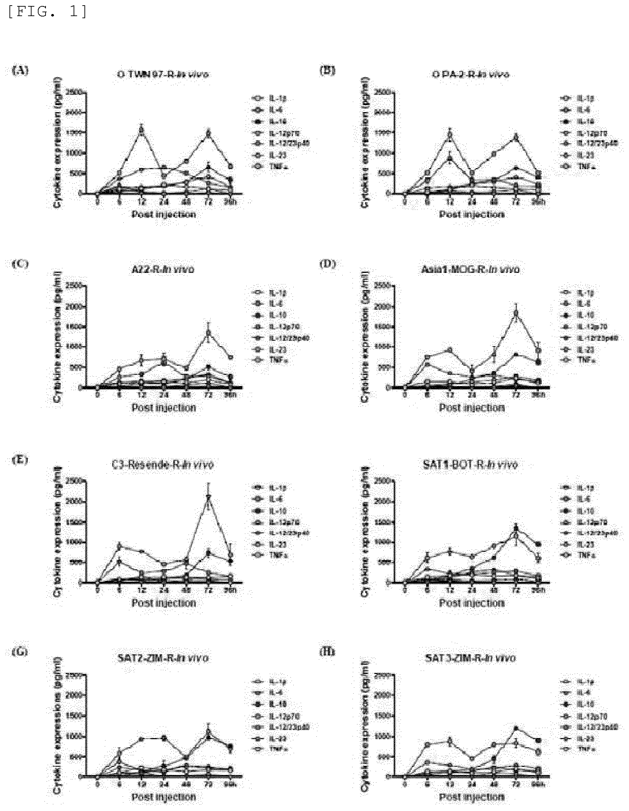

As a result of examining the cytokine expression patterns and transient changes due to the serotype-specific antigens, expressions of pro-anti-inflammatory cytokine IL-23 and anti-inflammatory cytokine IL-10 were highly demonstrated to competitively induce "cytokine storm" by most of the antigens. In the groups injected with SAT1-BOT-R, SAT2-ZIM-R and SAT3-ZIM-R, the expression of the above both cytokines were similarly observed from 48 hours to 72 and 96 hours, whereas O PA-2-R, O TWN 97-R, A22-R, C3-Resende-R and Asia1-MOG-R groups showed significantly higher expression of IL-23 than that of IL-10. IL-23 expression is characterized by similar expression patterns in the serotypes O, i.e., O PA-2-R and O TWN 97-R, that is, the expression rapidly increased at 6 to 12 hours and then showed a slightly low level at 24 hours. Then, the expression was re-boosted at 48 to 72 hours and approached the level at the time of 12 hours, and then significantly decreased at 96 hours (FIG. 1).

1-2. In order to investigate the innate and cellular immune responses in DCs and MΦs by serotype-specific FMDV antigens, DCs and MΦs were separated from naive mice and treated with respective antigens, followed by analysis of inflammatory cytokines such as IL-1α, IL-6, IL-10, IL-12p70, IL-12/23p40, IL-23 and TNFα in cell culture supernatant, anti-inflammatory cytokine expression patterns and time kinetics of expression of the cytokines after 0, 12, 24, 48, 72 and 96 hours. As a result, the expression in DCs is as follows.

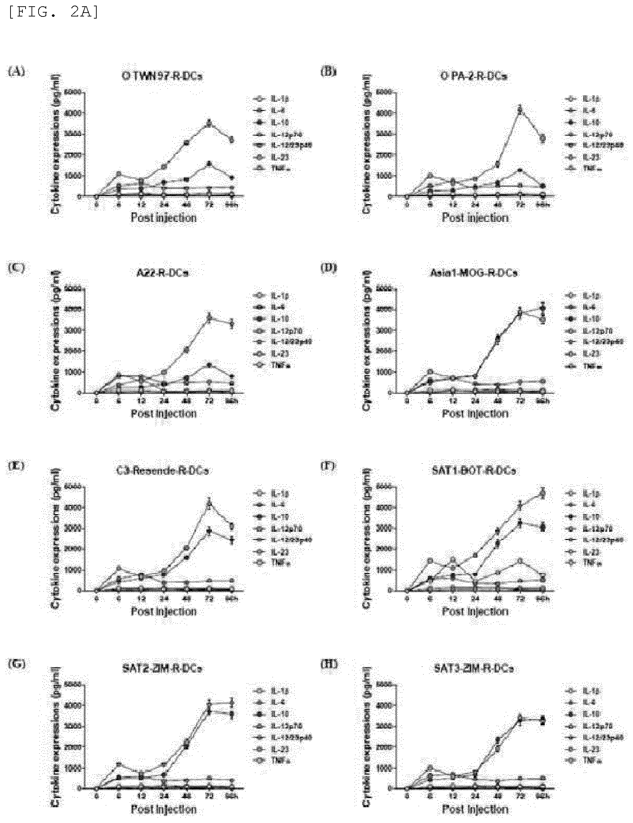

With regard to the expression level of IL-23 and IL-10 with respect to serotype, expression levels of the cytokines in SAT1-BOT-R, SAT2-ZIM-R, SAT3-ZIM-R, C3-Resende-R and Asia1-MOG-R treatment groups were substantially similar to one another. On the other hand, in the case of A22-R, O PA-2-R and O TWN 97-R, the expression level of IL-23 was significantly higher than that of IL-10. Further, the maximum expression level of IL-6 was high such as a range of 593 to 753 pg/mL, and the rest of the cytokines showed about the expression levels of 50 to 200 pg/mL. In most antigen-treated groups, the expression levels of IL-23, IL-10 and IL-6 crossover with each other at 12 hours, and the expression of IL-6, which was initially increased, showed a tendency to be down-controlled by the anti-inflammatory cytokine IL-10, whereas IL-23 showed a tendency to be continuously up-controlled (FIG. 2A).

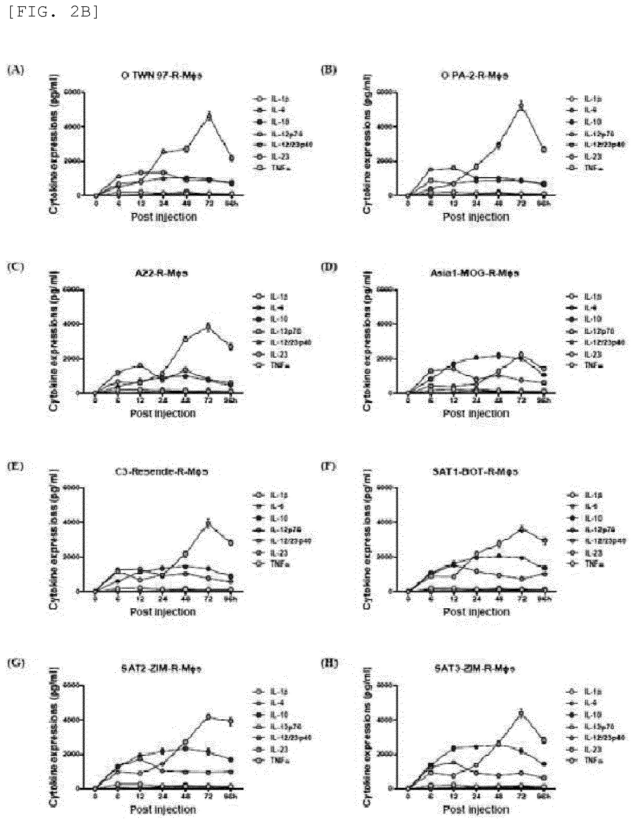

Overall cytokine expression in MΦs was similar to that in DCs, as shown in FIG. 2A. Further, with regard to a change in expression of cytokine to serotype, it was observed that IL-23 expression levels in most antigen-treated cells except for Asia1-MOG-R were significantly higher after 48 hours than IL-10. In the case of SAT1-BOT-R, SAT2-ZIM-R, SAT3-ZIM-R and C3-Resende-R treatment groups, the expression level was high in the order of IL-10 > IL-6 > IL-23 till 12 hours. However, after crossover of the expression levels of the above cytokines at 12 to 48 hours, the expression pattern was changed in the order of IL-23 > IL-10 > IL-6 after 48 hours. On the other hand, in the case of A22-R, O PA-2-R and O TWN 97-R, cytokine expression levels were high in the order of IL-6 > IL-23 and IL-10 till 12 hours, and after crossover of the expression levels of these cytokines at 12 to 24 hours, the expression pattern was changed in the order of IL-23 > IL-6 and IL-10 after 24 hours (FIG. 2B).

From the above results, it was confirmed that DCs and MΦs are effectively stimulated at the early stage when serotype-specific antigen treatment was performed. Further, it was demonstrated that inflammatory cytokines, that is, IL-23 and IL-6 and the anti-inflammatory cytokine, that is, IL-10 are mainly expressed from the stimulated cells.

### 2. Measurement of changes in antigen-mediated cytokines in vivo

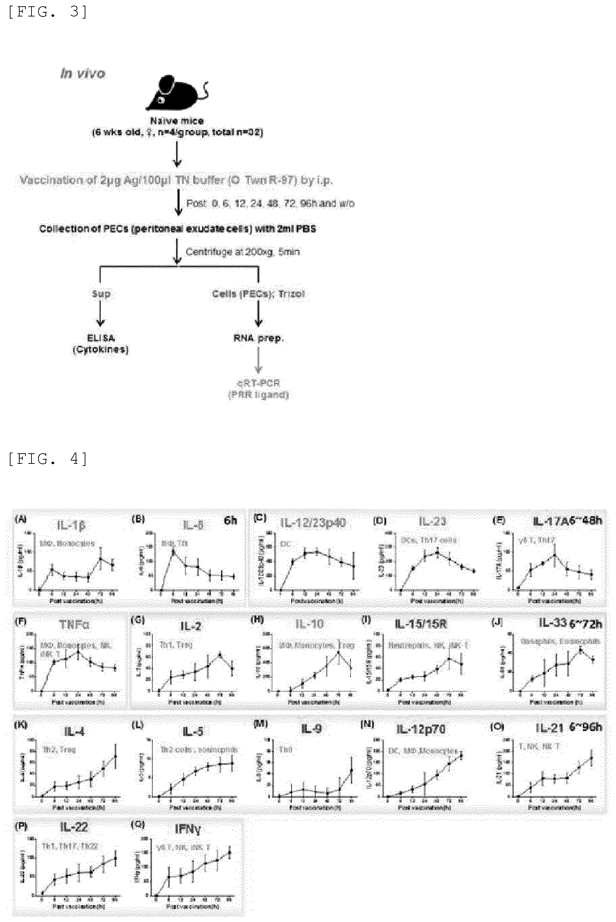

This test method is the same as shown in FIG. 3, and it was attempted to confirm stimulation of immune cells by measuring a change in cytokines.

As a result of the experiment, IL-1β (IL-1 beta) and IL-6 were initially reacted, and were mostly stimulated over time. IL-12/23p40, IL-17A and TNFα (TNF alpha) were stimulated between 12 and 24 hours, while IL-1β (IL-1 beta), IL-2, IL-10, IL15/15R and IL-33 were completely stimulated by 72 hours. Further, in the case of IL-4, IL-9, IL-12p70, IL-21, IL-22 and IFNγ (IFN gamma), the response was increased till 96 hours. Since the cytokines to be stimulated in cells are different, which cells work can be presumed (FIG. 4).

### 3. Measurement of change in antigen-mediated in vitro cytokine expression profiles of immune cells isolated from mouse peritoneal exudate cells (PECs) and splenocytes

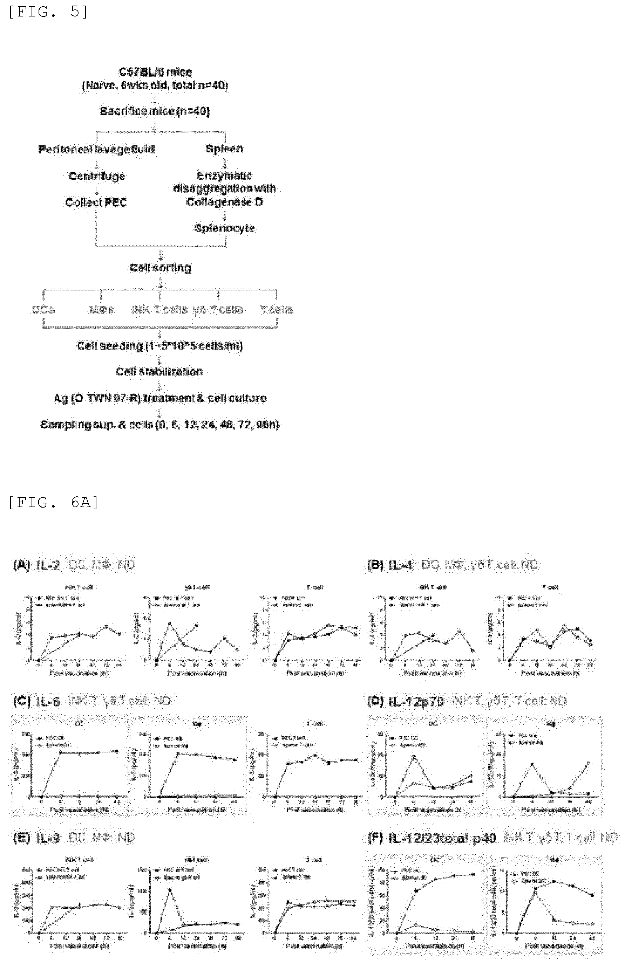

This test method is the same as shown in FIG. 5. Immune cells (DCs, MΦs, iNK T cells, γδ T cells and T cells) were isolated from mouse peritoneal exudate cells (PECs) and splenocytes, followed by measurement of a change in cytokine expression in order to confirm stimulation of specific immune cells. Specifically, cells were treated with O TWN 97-R antigen (2 µg), and the cells (DCs, MΦs, iNK T cells, γδ T cells and T cells) were isolated from the PECs and spleens, followed by measurement of changes over time (0, 6, 12, 24, 48, 72, 96 h).

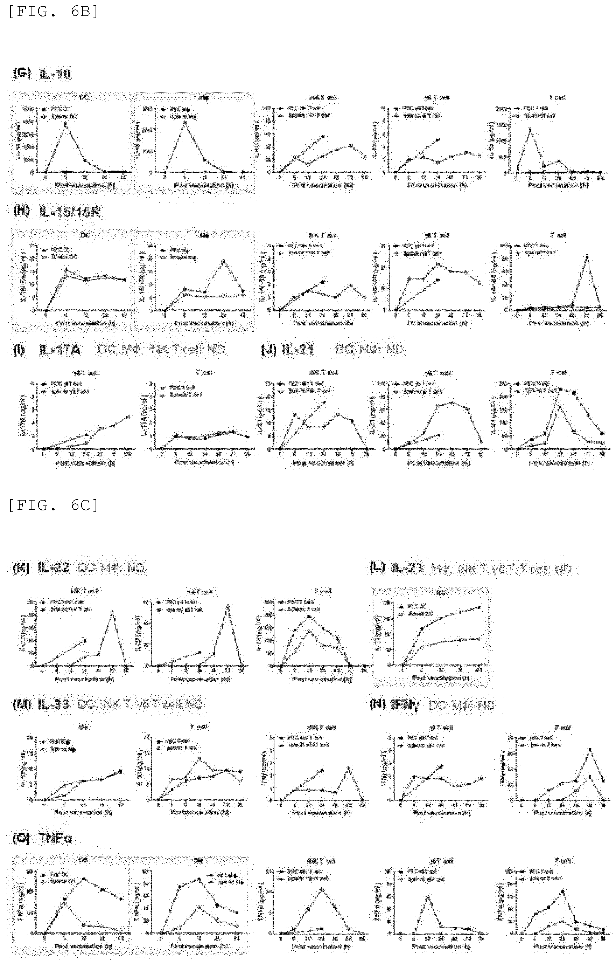

As a result of the experiment, reactivity of the cytokines IL-12p70, IL12/23 total p40, IL-10, IL-5 and TNFα, which are cytokines reacting with DCs, tended to be high in the initial 6 to 12 hours. Further, it could be confirmed that IL-12/23 p40 and IL-23 had a tendency of increased expression at 24 to 48 hours (FIGS. 6A to 6C).

### 4. Measurement of in vitro changes in O TWN 97-R antigen-mediated cytokines expression profiles through simultaneous culture in sorted cells

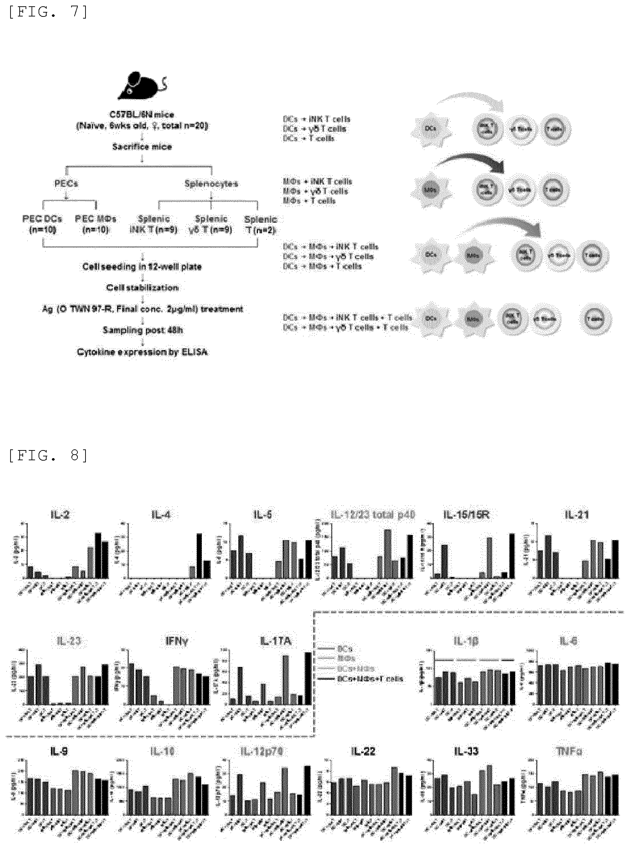

This test method is the same as in Preliminary Experimental Example 2 in order to sort cells, and immune cells (DCs, MΦs, iNK T cells, γδ T cells and T cells) were separated and mixed, followed by measurement of change in O TWN 97-R antigen-mediated cytokine in order to confirm stimulation of the immune cells (FIG. 7). Specifically, the cells were treated with O TWN 97-R antigen (2 µg), and results thereof were measured after 48 hours from antigen stimulation.

As a result of the experiment, it was confirmed that IL-2, IL-4, IL-5, IL-12/23 p40, IL-15/15R, IL-21, IL-23, IFNγ, etc. showed reactivity only when DCs were added. On the other hand, it was confirmed that IL-β, IL-6, IL-9, IL-10, IL-12p70, IL-17A, IL-22, IL-33, TNFα, etc. showed reactivity even without addition of DCs (FIG. 8).

### 5. Analysis of defense mechanism of antigen stimulation through innate immune response

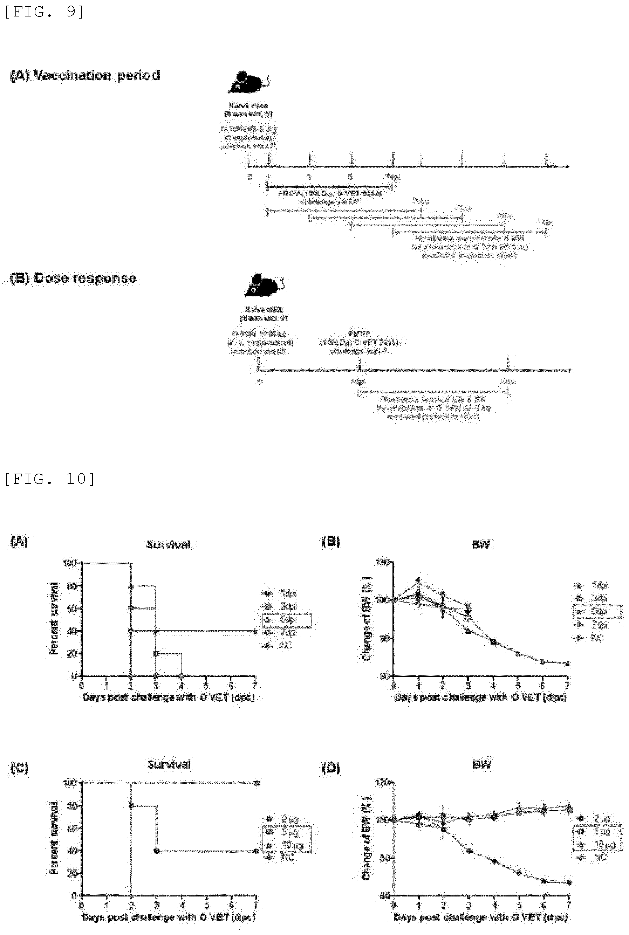

5-1. A research strategy for confirming whether the O TWN 97-R antigen, which activates the innate immune response and the cellular immune response, actually exhibits defense effects upon infection with the FMD virus is shown in FIG. 9. First, in order to determine a vaccination period of the antigen, 2 µg of the antigen was injected into the mouse intraperitoneally, the mouse was infected with virus at 1, 3, 5, and 7 dpi, and then a survival rate and change of body weight thereof were monitored till 7 dpc. Thereafter, in order to confirm dose-specific response of the antigen, 2, 5, and 10 µg of antigens were injected into the same route, the mouse was infected with virus at 5 dpi, and the survival rate and change of body weight were monitored till 7 dpc.

As a result, the 5 dpi group showed a 40% defense rate, while the group with a dose increasing to 5, 10 µg showed a 100% survival rate without weight loss (FIG. 10).

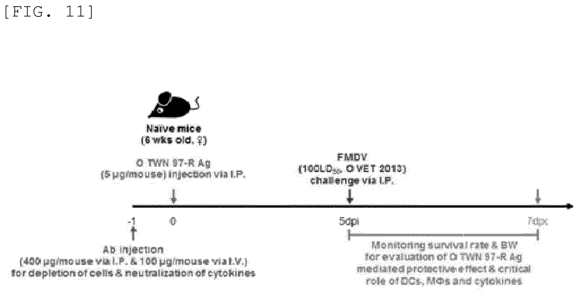

5-2. In defending the host from foot-and-mouth disease infection, it was attempted to confirm which cells among DCs and MΦs are important, or which cytokines exhibit important effects (FIG. 11).

Antibodies were used to deplete cells or neutralize cytokines, and antigens were injected 24 hours later, followed by infection with virus 5 days later (5 dpi) to confirm defense performance. As a result, it was confirmed that IL-23p19, TNFα and IL-10 play an important role. All mice died on day 5 (5 dpc) in the group administered with the antibody against the inflammatory cytokine IL-23p19, and all mice died on day 6 (6 dpc) in the group administered with the TNFα antibody, whereas the group administered with the antibody against anti-inflammatory cytokine IL 10 showed a 100% survival rate and no weight loss was observed. In antigen-induced cytokine neutralization experiments by specific antibodies, with regard to IL-23, TNFα, IL-1β, IL-6 and IL-10 identified to be important in defending the host from FMDV attack in the experiment, defense effects by these cytokines, respectively, were confirmed. First, each cytokine was intraperitoneally injected into a mouse in an amount of 5 µg/100 µL PBS and the mouse was infected with O VET (ME-SA) 6 hours later, followed by monitoring the survival rate and change of body weight till 7 dpc. On the other hand, a negative control group administered with 100 µl PBS in the same route was used for comparison. As a result, after injection of IL-23, it was confirmed that the survival rate of mice in the group infected with FMDV was 100% to demonstrate complete protection. Further, no change in the body weight was observed as compared to before infection.

In the case of IL-1α and TNFα treatment groups, the survival rate was low. Further, although the body weight was reduced by about 30%, mortality tended to be delayed. On the other hand, in the case of the IL-6 and IL-10 treatment groups, mice died in the early stage of infection to demonstrate no defense effect.

From the above results, it was considered that the cytokine IL-23 plays the most important role in host defense against FMDV attacks. IL-1α and TNFα are also weaker than IL-23, but these were determined to partially contribute to the host defense at the beginning of virus infection.

On the other hand, in the case of IL-10 as an anti-inflammatory cytokine, IL-10 and antigen were simultaneously injected at 5 µg as well as injection of IL-10 alone, in order to compare the survival rates and change of body weight therebetween.

In order to suppress IL-23, IL-1α, IL-6 and TNFα, which are expressed at high levels by antigens, each of these cytokines was neutralized with a specific mouse monoclonal antibody, and IL-10 and antigen were simultaneously injected followed by virus infection at 24 hours. Isotype control antibody for each antibody was injected and used as a control group for comparison. As a result, similar to the IL-10 treatment group, the survival rate was decreased to 20% on day 4 after FMDV infection (4 dpc), all animals died on day 6 (6 dpc), and the change of body weight was also reduced by about 30%. Therefore, it seemed that IL-10 inhibited the immune response within the host at the early stage of FMDC infection, so as to increase susceptibility to viruses, thereby deteriorating host defense.

From the above results, it is believed that, if expression of IL-23 as an inflammatory cytokine can be increased at the early stage of FMDV infection while inhibiting production of IL-10, *in vivo* cellular immune response may be activated, thereby more efficiently defending the host.

Further, in order to confirm whether IL-23 exhibits immune-stimulatory activity as an adjuvant (vaccine adjuvant), 2.5 µg of IL-23 was administered in combination with the same amount of antigen.

As a result, the mice in the group administered with 2.5 µg of antigen alone showed a survival rate of 40%, whereas the group administered with IL-23 and antigen together had a survival rate of 80% or more, and weight loss was insignificant on day 2 to 3 (2 dpc) and then showed a tendency of recovering again. Therefore, it was confirmed that defense effects on FMDV infection were improved by adjuvant effects of IL-23.

On the other hand, in order to confirm whether DCs and MΦs stimulated by antigen actually play an important role in host defense at the beginning of FMDV infection, the activated DCs and MΦs were separated and transplanted into naive mice 6 hours after antigen administration, followed by virus infection 1 hour later. Then, the survival rate and change of body weight were observed till 7 dpc.

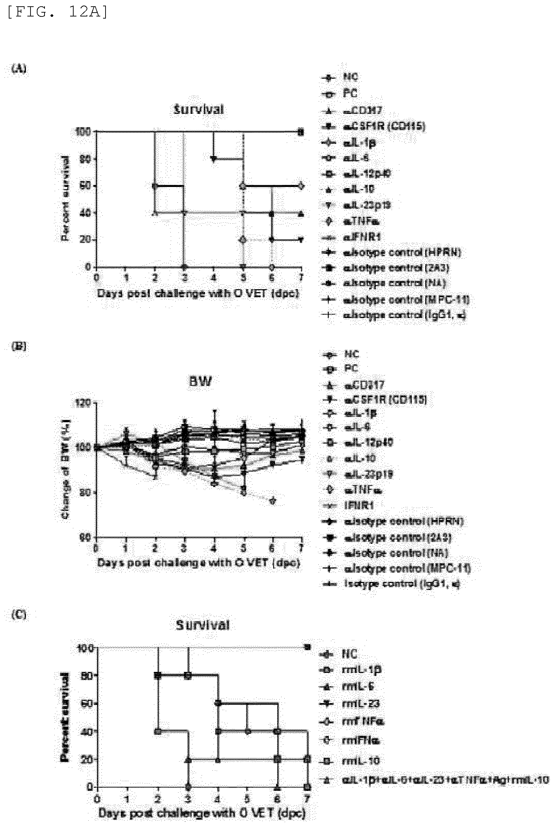

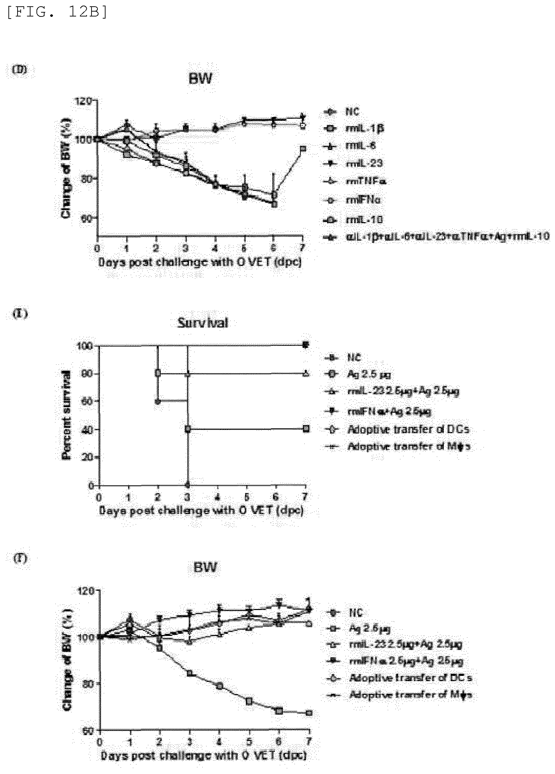

As a result, it was confirmed that all mice survived 100% in the activated DCs and MΦs transplantation groups, and no body weight change was also observed (FIGS. 12A and 12B) .

From the above results, it is expected that stimulation of DCs and MΦs by antigens promotes secretion of inflammatory cytokines, chemokines and co-stimulatory molecules from these cells at the beginning of FMDV infection, and plays an important role in defending the host by presenting antigens to T cells.

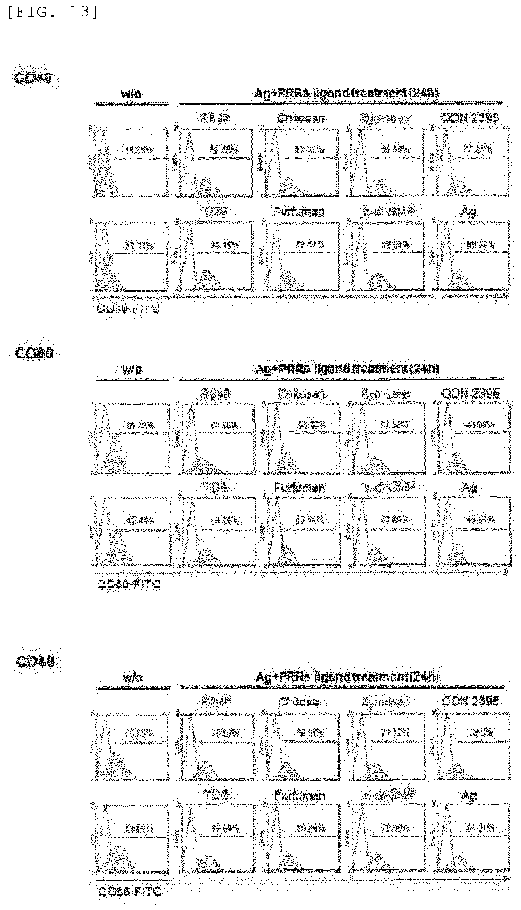

The results of examining expression of antigen-mediated co-stimulatory molecules in DCs are as follows (FIG. 13). As a result of examining expression of CD40, CD80 and CD86 after treatment with an antigen alone or by adding the PRRs ligand to the antigen as an adjuvant, high expression was observed in the treatment groups with addition of R848, Zymosan, TDB and c-di-GMP, respectively. Therefore, although DCs are stimulated with the antigen alone to increase the expression co-stimulatory molecules, it is expected that using PRRs ligand as an adjuvant for treatment may stimulate DCs more strongly, thus effectively presenting the antigen to T cells.

Previously, changes in O TWN 97-R Ag-derived cytokines were observed in DCs and MΦs. In order to investigate which PRRs ligand signal initiates expression of these cytokines, DCs and MΦs were separated from PECs of naive mice and treated with O TWN 97-R Ag. Then, 6 hours after the treatment, cells were sampled and RNA was prepared, followed by confirming a gene expression level of the PRRs ligand through qRT-PCR.

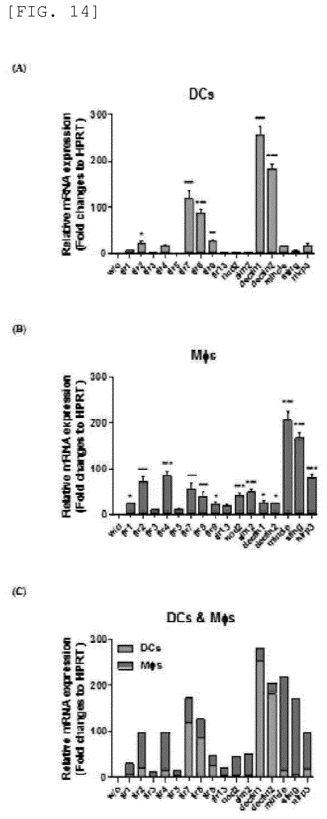

Consequently, as shown in FIG. 14, expression of *dectin1, dectin2, tlr7,* and *tlr8,* etc. was high in DCs, whereas expression of *mincle* and *sting* was high in MΦs, and expression of *tlr2, tlr4, nlrp3,* etc. was also relatively high. When looked at the overall gene expression levels in DCs and MΦs, PRRs ligand gene expression level was high in the order of *dectinl> mincle> dectin2> tlr7>* sting> *tlr8.*

From the above results, it could be understood that O TWN 97-R Ag stimulates cell-intrinsic PRRs ligands such as *tlr7, tlr8, sting,* etc., as well as cell-extrinsic PRRs ligands such as *dectinl, dectin2, mincle,* etc. simultaneously, thereby amplifying innate immune response in APCs such as DCs and MΦs, and cellular immune response. Further, a differentiation process of Th cells into Th1, Th2 or Th17 cells may be promoted, thereby eventually contributing to activation of T cells. Consequently, it is expected that the above substance may induce a cellular immune response and play a decisive role in host defense at the early stage of FMDV infection.

### <Example 1> Screening of adjuvant candidates through efficacy assessment in mice

### 1-1. Potential of PRRs ligand and cytokine as vaccine adjuvants and analysis of memory effects

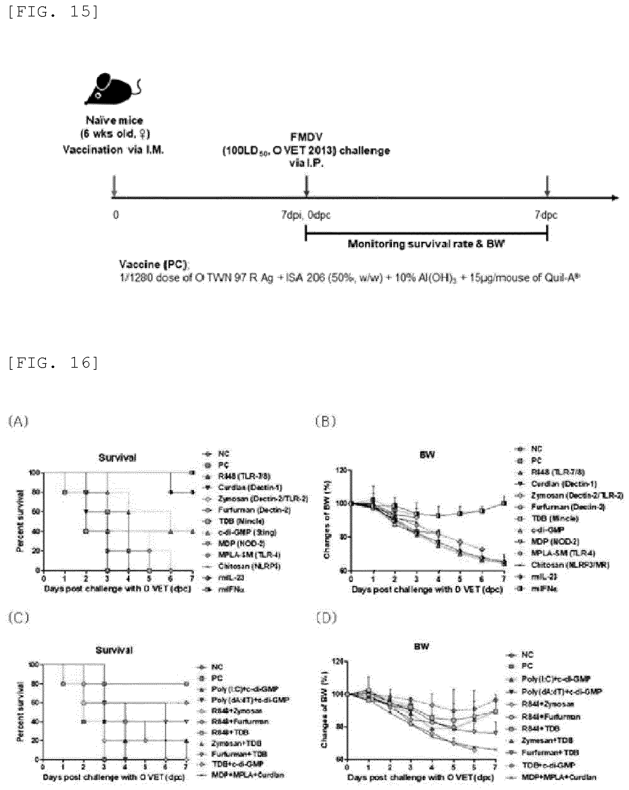

In order to assess the potential of PRRs ligand and recombinant cytokine as vaccine adjuvants and memory effects on FMDV infection, the materials of Table 1 were tested as shown in FIG. 15.

### [TABLE 1]

Experimental design for assessment of PRRs ligands and cytokines-mediated adjuvanticity on mice

| Group | Vaccine combination | Adjuvant | Dose |
|---|---|---|---|
| | | PRRs ligand or cytokine | |
| NC | - | - | - |
| PC | O TWN 97-R | - | - |
| PC+ Adju vant | 11.7ng (1/1280 dose for cattle or pig use) of O TWN 97 R Ag+ ISA 206 (50%, w/w) +10%Al(O H) 3 +15 µgQuil-A^{®} | R848 (TLR-7/8) | 100 µg |
| | | Curdlan (Dectin-1) | 45 µg |
| | | Zymosan (Dectin-2/TLR-2) | 50 µg |
| | | Furfurman (Dectin-2) | 50 µg |
| | | TDB (Mincle) | 50 µg |
| | | c-di-GMP (Sting) | 50 µg |
| | | MDP (NOD-2) | 30 µg |
| | | MPLA-SM (TLR-4) | 20 µg |
| | | Chitosan (NLRP3 inflammasome inducer, MR) | 100 µg |
| | | rmIL-23 | 5 µg |
| | | rmIFNα | 2.5×10⁶IU |
| | | Poly(I:C) (TLR-3/MDA-5)+c-di-GMP (Sting) | 50 µg+50 µg |
| | | Poly(dA:dT) (RIG-I/CDS agonist)+c-di-GMP (Sting) | 25 µg+50 µg |
| | | R848 (TLR-7/8)+Zymosan (Dectin-2/TLR-2) | 50 µg+25 µg |
| | | R848 (TLR-7/8)+Furfuman (Dectin-2) | 50 µg+25 µg |

| | | | |
|---|---|---|---|
| | | R848 (TLR-7/8)+TDB (Mincle) | 50 µg+25 µg |
| | | Zymosan (Dectin-2/TLR-2)+TDB (Mincle) | 25 µg+25 µg |
| | | Furfuman (Dectin-2)+TDB (Mincle) | 25 µg+25 µg |
| | | TDB (Mincle)+c-di-GMP (Sting) | 25 µg+25 µg |
| | | MDP (NOD-2) +MPLA (TLR-4) +Curdlan (Dectin-1) | 30 µg+20 µg+45 µg |

When the PRRs ligand and cytokine were administered alone, the survival rate was high in the order of IFNα > IL-23 > c-di-GMP administration groups, as shown in FIG. 16(A). Further, in the case of IFNα in change of body weight of FIG. 16(B), no body weight change was substantially observed. Further, FIG. 16(C) shows the survival rate when administered in combination with the PRRs ligand.

The survival rate was high in the order of R848+TDB > TDB+c-di-GMP > Furfurman+TDB, while observing only weight loss in the range of less than about 20% in terms of weight change (FIG. 16(D)).

From the above results, it could be seen that, when administered in combination with cytokines compared to administration of PRRs ligand alone, mice were more effectively protected from FMDV infection. In particular, combinations of TLR-7/8, Mincle, Dectin-2, Sting, etc. were determined to have higher potential as vaccine adjuvants.

### 1-2. PRRs ligand and cytokine-mediated memory immune response in mice

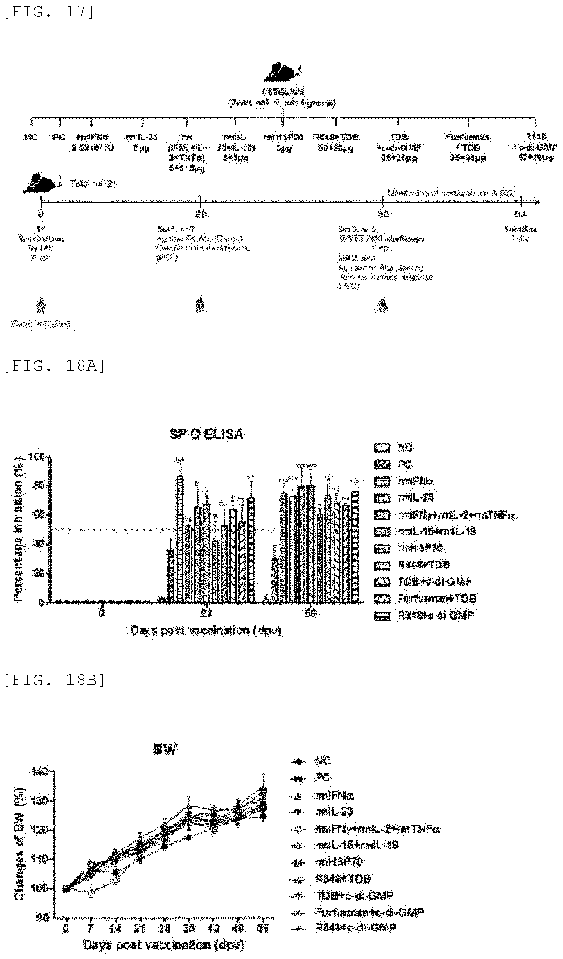

In order to assess the potential of the PRRs ligand and the recombinant cytokine as vaccine adjuvants and the memory effects on FMDV infection, an experiment was performed according to the strategy as shown in FIG. 17. Foot-and-mouth disease antigen used herein was O TWN 97-R, and the vaccine composition of a positive control (PC) group was as follows; O TNW 97-R Ag (15 µg/dose/ml, 1/160 dose), ISA 206 (50%, w/w), 10% Al(OH)₃, 15 µg/mouse of Quil-A.

Mice were subjected to 1^{st} vaccination with I.M. (intramuscular inoculation) at 0 dpv (days post vaccination), followed by 2^{nd} vaccination (boosting) 28 days later (28 days post vaccination, dpv). Thereafter, on day 56 (56 days post vaccination, dpv), FMDV (100LD₅₀, of O VET 2013, ME-SA topotype) was administered intraperitoneally to the mouse, followed by monitoring the survival rate and change of body weight till 7 days (days post challenge, dpc). Further, in order to confirm an ability of inducing cellular and humoral immune responses, a mouse serum and peritoneal exudate cells (PECs) were sampled at 0, 28, and 56 dpv, which in turn were used in analysis such as SP ELISA and virus neutralization titers (VN titers, neutralizing antibodies).

As a result of the experiment, as shown in FIG. 18A, IFNα, IFNγ+IL-2+TNFα, IL-15+IL-18, TDB+c-di-GMP and R848+c-di-GMP administration groups at 28 dpv showed significantly increased antibody titers as compared to the control group. Further, at 56 dpv, antibody titers were significantly higher in all cytokines and PRRs ligand adjuvant administration groups. In particular, in the case of IFNα, antibody titers were maintained at a high level till 56 dpv. In the case of the IL-23-administered group, which had an excellent increase in cellular immune response in the previous experiments, the antibody titer was also observed to be high, thereby indicating that humoral immune responses may be effectively induced.

Meanwhile, even in the case of IFNγ+IL-2+TNFα-administered group, which is involved in T cell activity and T cell-mediated cellular and humoral immune responses, the antibody titer remained steadily high after 28 dpv, while the group administered with IL-15+IL-18 involved in mucosal immunity as an adjuvant also exhibited high antibody titers. Further, when HSP70 known to be involved in long-term immunity was administered, it was observed that the antibody titer was relatively increased to a high level although it was slightly lower than those of other adjuvant-administered groups. Further, in the case of PRRs ligand administration groups, high antibody titers were shown in all groups.

In order to observe the change of body weight due to vaccination itself, the change of body weight was monitored once a week for 8 weeks (56 dpv) after vaccination (FIG. 18B). As a result, the group administered with a combination of IFNγ+IL-2+TNFα had a slight weight loss at 7 dpv, however, there was no significant difference. Further, this result is presumed because the dose of recombinant cytokines was slightly higher (total 15 µg) than those of other groups (total 5 to 10 µg).

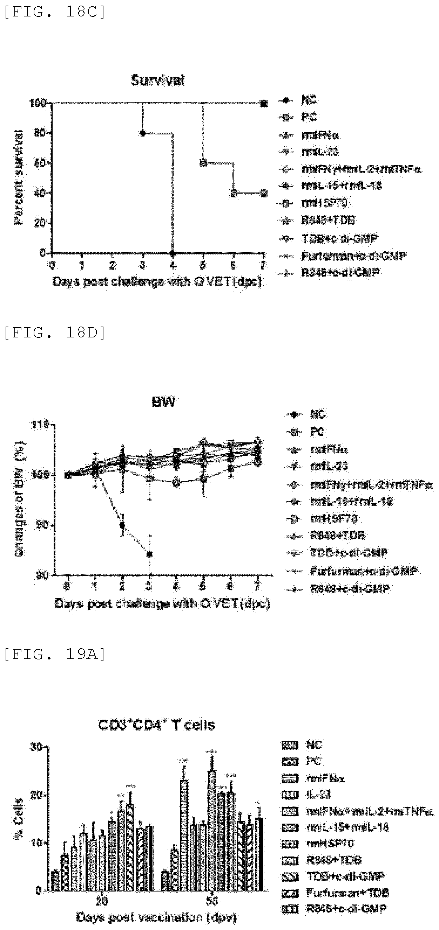

On day 56 after vaccination (56 dpv), as a result of observing the survival rate (FIG. 18C) and body weight (BW) change (FIG. 18D) after infection with O VET 2013, mice in the negative control group died 100% on day 4, while mice in the positive control group showed a tendency to survive 40%. On the other hand, mice in the group administered with PRRs ligand and cytokine adjuvant survived 100%, while observing no body weight loss.

Therefore, when these cytokines and PRRs ligands were used as adjuvants, it was expected that both a vaccine-induced cellular immune response and a humoral immune response are effectively induced to contribute significantly to the host defense.

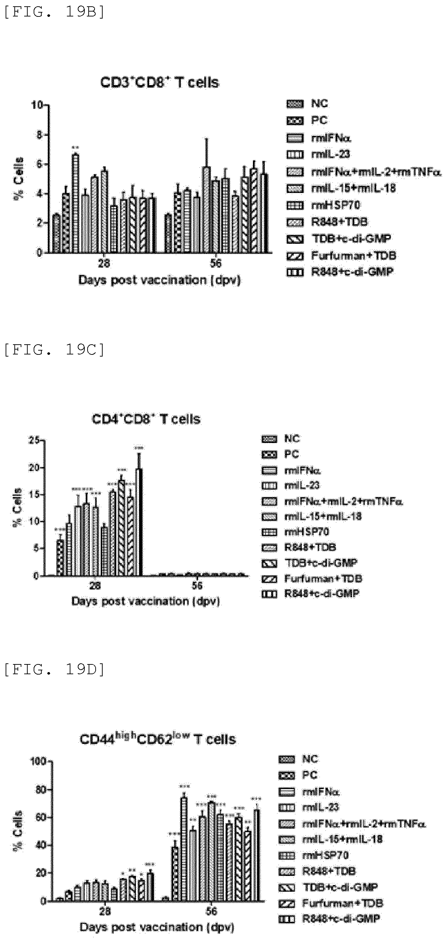

In order to observe the induction of cellular and humoral immune responses by cytokines and PRRs ligands, expansion of cells involved in these immune responses was analyzed by FACS. In FIG. 19A, the number of CD3⁺CD4⁺ T cells was confirmed, and expansion of cells was observed in the groups to which IFNα, HSP70, R848+TDB, TDB+c-di-GMP, etc. were added. FIG. 19B shows a population of CD3⁺CD8⁺ T cells. Compared to CD4⁺ T cells, the overall number of cells appeared to be lower, and the IFNα-treated group exhibited significance at 28 dpv.

In FIG. 19C, expansion of CD4⁺CD8⁺ T cells was confirmed. At 28 dpv, all cytokines and PRRs ligand addition groups except HSP70 showed a significant increase in cell number while having a tendency of considerably decreased number at 56 dpv. As a result of observing the expansion of CD44^{high}CD62^{low} T cells, which are known as a marker of memory T cells (FIG. 19D), the population was rapidly increased at 56 dpv compared to 28 dpv. Further, PRRs ligands induced memory T cell expansion more significantly than cytokines at 28 dpv, whereas the number of cells was increased in all adjuvant-added groups at 56 dpv. Therefore, these adjuvants are expected to effectively induce memory T cells so as to promote stimulation of the cellular and humoral immune responses.

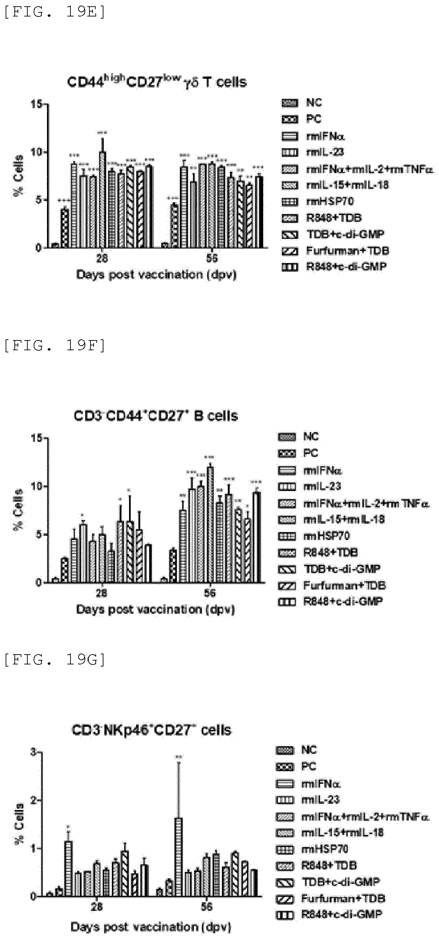

In FIG. 19E, expression of CD44^{high}CD27^{low} γδ T cells, which are memory γδ T cells, was observed. It is known that about 5% of γδ T cells exist in mice, but the number of these cells was increased by about 8 to 10% due to addition of the adjuvant while no significant difference between 28 and 56 dpv was observed. Therefore, it could be seen that the addition of adjuvant may be effective not only for induction of initial cellular immune response but also for induction of mid-term and long-term cellular immune responses.

FIG. 19F shows the expansion of memory B cells (CD3⁻ CD44⁺ CD27⁺ B cells). At 28 dpv, IL-23, R848+TDB, TDB+c-di-GMP showed a significant increase in the number of cells, and the number of cells was generally increased at 56 dpv compared to that at 28 dpv, and memory B cells in all cytokines and PRRs ligand added groups were confirmed to be significantly expanded. In particular, the IL-23, IFNγ+IL-2+TNFα, IL-15+IL-18, R848+TDB and R848+c-di-GMP added groups showed a significance of p<0.001 or less.

FIG. 19G shows the results of confirming the expansion of CD3⁻CD335 (NKp46)⁺CD27⁺ cells known as memory like NK cells. Specifically, the IFNα-administered group showed a significantly high number of cells, and it is considered from the result that IFNα appears to effectively stimulate NK cells involved in the innate immune response, and excellent effects on induction of a memory response may be achieved.

### 1-3. Potential of cytokine as an adjuvant and protective effects on FMDV infection by various serotypes in mice

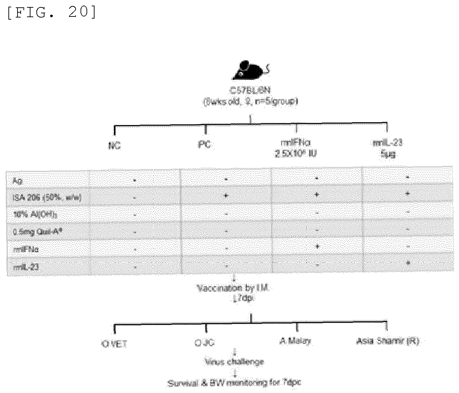

In order to observe the potential of cytokines, in particular, IFNα and IL-23 as vaccine adjuvants, memory responses, and defense effects on FMDV infection by various serotypes, animal experiments as shown in FIG. 20 were performed.

To confirm the above effects, O VET (O Vet 2013), O JC (O Jincheon 2014), A Malay (A Malay 97), Asia Shamir-R (a whole genome recombinant clone of Asia1 Shamir) and PC groups were injected with only ISA 206 oil immunity enhancer, and NC was a negative control group.

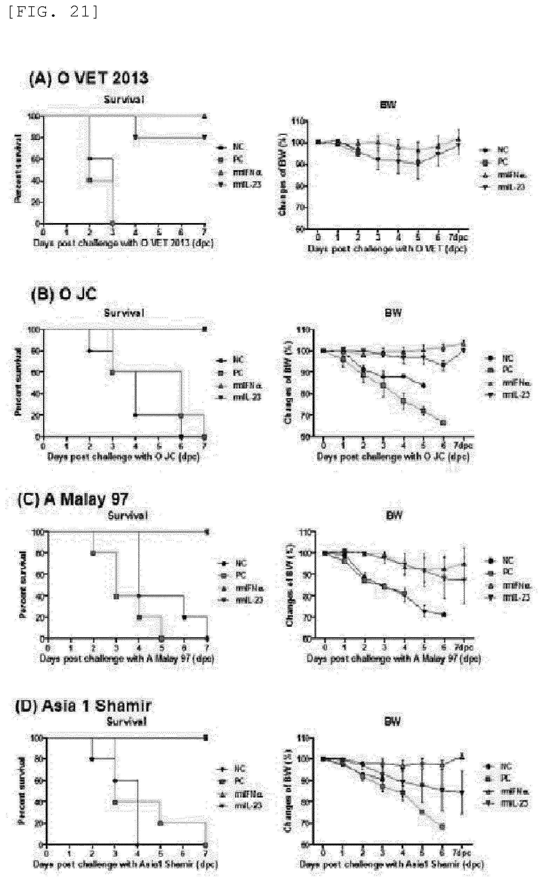

As a result of the experiment, the group administered with both of IFNα and IL-23 as an adjuvant almost exhibited complete defense against attacks of O VET2013, O JC, A Malay 97, and Asia1 Shamir, while a change of body weight was very little reduced compared to before virus attack (FIG. 21).

Therefore, the memory function of IFNα and IL-23 *in vivo* was confirmed, and it was expected that the above substances may effectively defend the host against FMDV attack by various serotypes regardless of the specificity between antigen-virus contained in the vaccine.

### <Example 2> Assessment of reactivity of adjuvant candidates by livestock species

Based on the experimental results in regard to the above mice, experiments were performed using the adjuvant composition of Table 2 below so as to observe PRRs ligand, HSP70 and cytokine-mediated cell proliferation and cytotoxicity in bovine and porcine immune cells.

**[TABLE 2]**

| **Gro up** | **Adjuvant** | **Dose (µg/ml)** | **Gro up** | **Adjuvant** | **Dose (µg/ml )** |
|---|---|---|---|---|---|
| 1 | Con | | 13 | Poly(I:C) (TLR-3)+c-di-GMP (Sting) | (5 µg+2 µg) /ml |
| 2 | R848 (TLR-7/8) | 10 µg/ml | 14 | Poly (dA:dT) (RIG-I&CDS agonist) + c-di-GMP (Sting) | (5 µg+2 µg) /ml |
| 3 | Curdlan (Dectin-1) | 100 µg/ml | 15 | R848(TLR-7/8)+Zymosan (Dectin-2/TLR-2) | (5 µg+5 µg) /ml |
| 4 | Zymosan (Dectin-2/TLR-2) | 10 µg/ml | 16 | R848(TLR-7/8)+Furfuman(Dect in-2) | (5 µg+5 µg) /ml |
| 5 | Furfurman(Dectin -2) | 10 µg/ml | 17 | R848 (TLR-7/8)+TDB (Mincle) | (5 µg+5 µg) /ml |
| 6 | TDB (Mincle) | 10 µg/ml | 18 | Zymosan (Dectin-2/TLR-2)+TDB(Mincle) | (5 µg+5 µg) /ml |
| 7 | c-di-GMP (Sting) | 4 µg/ml | 19 | Furfurman(Dectin-2)+TDB (Mincle) | (5 µg+5 µg) /ml |
| 8 | MDP (NOD-2) | 10 µg/ml | 20 | TDB (Mincle) +c-di-GMP (Sting) | (5 µg+2 µg) /ml |
| 9 | MPLA-SM (TLR-4) | 1 µg/ml | 21 | MDP (NOD-2)+MPLA-SM (TLR-4)+Curdlan (Dectin-1)) | 10 µg+1 µg+100 µg/ml |
| 10 | Chitosan(NLRP3 inflammasome, MR) | 100 µg/ml | 22 | Oil (ISA206) | 25% |
| 11 | Poly(I:C) | 10 µg/ml | 23 | Gel (Alum) | 10% |
| 12 | Poly(dA:dT) | 10 µg/ml | 24 | Saponin (Quil-A) | 2.5 µg/ml |
| | | | 25 | Oil(ISA206)+Gel(Al um)+Saponin (Quil-A) | (25%+1 0%+2.5 µg) /ml |

### Isolation of PBMCs

Whole blood from pigs and cattle was donated by the Animal Sanitation Laboratory in Gyeonggi Province. 15 ml of whole blood was collected using a BD Vaccutainer heparin tube (BD, Becton, Dickinson and Company, Franklin Lakes, NJ, USA), and peripheral blood mononuclear cells (PBMCs) were collected by means of Ficoll-Paque^{™} PLUS (GE Healthcare Bio-Sciences Corp., Piscataway, NJ, USA) density gradient centrifugation. The residual red blood cells were lysed by treatment with ACK (Ammonium-Chloride-Potassium) Lysing Buffer (Gibco, Carlsbad, CA, USA).

The solution was suspended in Ca²⁺ and Mg²⁺ (Gibco, Carlsbad, CA, USA)-free Dulbecco's PBS supplemented with 2% FBS (Gibco, Carlsbad, CA, USA), and the number of cells was counted by a volumetric flowmeter (MACSQuant Analyzer, Miltenyi Biotec, Bergisch Gladbach, Germany). All cells were freshly isolated immediately before use while cryo-preserved cells were not used.

Purified PBMCs were re-suspended in RPMI-1640 (Gibco, Carlsbad, CA, USA; St. Louis, Missouri) supplemented with 10% fetal bovine serum (FBS) (HyClone, Logan, Utah, USA), 3mM L-glutamine (Sigma-Aldrich, USA) and 100 U/ml of penicillin-streptomycin (Sigma-Aldrich, St. Louis, MO, USA), followed by culture at 37 °C under 5% CO₂ and then adhesion of 1×10⁴ cells per well to a 96 well plate. After incubation for 3 hours, the culture medium was replaced with a serum-free medium before stimulation with various PRR ligands and cytokines.

### PRRs ligand and cytokine treatment

Porcine and bovine PBMCs were treated with PRR ligand and cytokines shown in Table 2. After 96 hours, the cell culture medium (supernatant) was collected, and cytotoxicity due to secretion of lactic acid dehydrogenase (LDH) and cell proliferation by incorporation of 5-bromo-2'-deoxyuridine (BrdU) were observed.

### Measurement of PRRs ligand and cytokine-mediated LDH secretion in porcine and bovine PBMCs

Levels of cytotoxicity in the supernatant of treated porcine and bovine PBMCs are as described above. LDH secretion analysis was performed according to the manufacturer's protocol using CytoTox 96 NonRadioactive Cytotoxicity Assay (Promega).

The percentage of LDH secretion was calculated as follows: percentage of LDH secretion = 100 × (absorbance reading of untreated control)/(absorbance of maximum LDH emission control - absorbance of non-absorbance control)

The cells were lysed using a lysis buffer in a kit and the supernatant from the lysis buffer-treated cells was used to determine the maximum LDH release control.

### BrdU incorporation analysis in porcine and bovine PBMC

A BrdU cell proliferation assay kit (Cell Signaling Technology, Beverly, MA, USA) was used to evaluate effects of PRRs ligands and cytokines on the proliferation of porcine and bovine PBMCs based on the incorporation of BrdU during DNA synthesis.

10 µM BrdU was added to the cell culture solution and incubated at 37 °C for 4 hours. Cells were then fixed and incubated with anti-BrdU mouse monoclonal antibody and horseradish peroxidase (HRP)-conjugated goat anti-mouse antibody. Chromogenic substrate tetramethyl benzidine was used to develop color. The absorbance was measured at a double wavelength of 450/550 nm.

### Vaccination and sampling

Experiments were conducted to observe the potential of PRRs ligand as an adjuvant, induction of cellular and humoral immune responses and long-term immune effects in pigs and cattle. As a foot-and-mouth disease antigen, O TWN 97-R was used, and the vaccine composition of the positive control group is as follows; O TNW 97-R Ag (15 µg/dose/ml), ISA 206 (50%, w/w), 10% Al(OH)₃, 150 µg/dose/ml Quil-A.

1 ml of the vaccine was administered twice (0 dpv, 28 dpv) at a 28-day interval through a deep intramuscular route of the animal's neck. Blood samples were collected at 0, 14, 28, 42, 56, 70, 84 dpv for pigs, and at 0, 14, 28, 56, 84, 112, 140, 168 dpv for cattle, respectively.

Animals were monitored daily for body temperature and appetite. Serum samples were stored at -80 °C until the test was performed.

### Serological test

### 1) ELISA (enzyme-linked immunosorbent assay) for detection of structural protein antibodies

In order to detect structural protein (SP) antibody in a serum, PrioCHECK FMDV type O (Prionics, Switzerland) was used. The absorbance of an ELISA plate was converted to a percentage of inhibition (PI) value. If the PI value was 50% or higher, the animal was considered antibody positive.

### 2) Virus neutralization test

Virus neutralization test was performed according to the OIE manual. The virus was activated by bathing in serum at 56 °C for 30 minutes. By adjusting a cell density to form a 70% monolayer, a 2-fold dilution of the serum samples from 1:4 to 1:512 was prepared. The diluted serum samples were incubated at 37 °C for 1 hour with 100 tissue culture infection dose (TCID) 50/0.5 ml homolog virus. After 1 hour, LF-BK (bovine kidney) cell suspension was added to all wells. After 2 to 3 days, CPE was measured to determine titer at which 100TCID₅₀ of the virus is neutralized by log 10 of a titer antibody dilution.

### 2-1. Results of reactivity assessment of adjuvant candidates in cattle

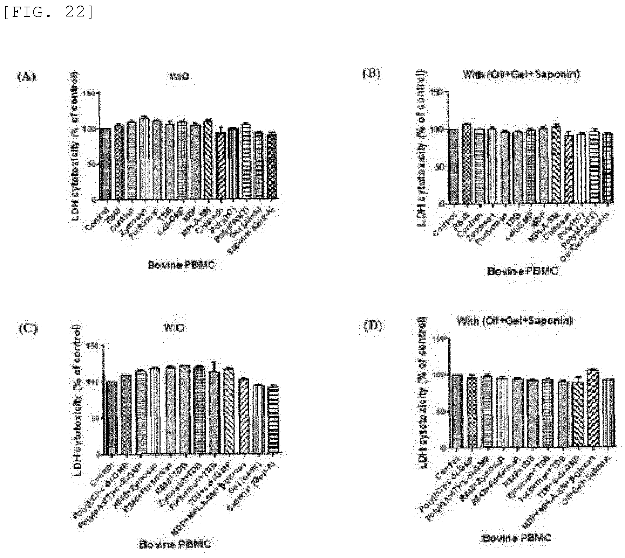

As a result of confirming the secretion of LDH (Lactate Dehydrogenase) to observe cytotoxicity due to PRRs ligand, cytokines, gel, saponin, etc., respectively, in bovine-derived PBMCs, the cytotoxicity of the treatment group itself was found to be low. Thus, there was no toxicity at the adjuvant concentration used in the present experiment (FIG. 22(A)). FIG. 22(B) shows the results of observing the cytotoxicity after treatment of the bovind-derived PBMCs with PRRs ligand and cytokines, respectively, mixed with the vaccine adjuvant Oil+Gel+Saponin. Even at this time, cytotoxicity due to the adjuvant mixture was not observed when compared to the control group.

On the other hand, cytotoxicity due to the above adjuvants was not observed even after combined treatment with the combination of PRRs ligands (FIG. 22(C)). Further, even when the PRRs ligands were combined, also mixed with the vaccine adjuvant Oil+Gel+Saponin and then used for treatment, cytotoxicity was not observed (FIG. 22(D)).

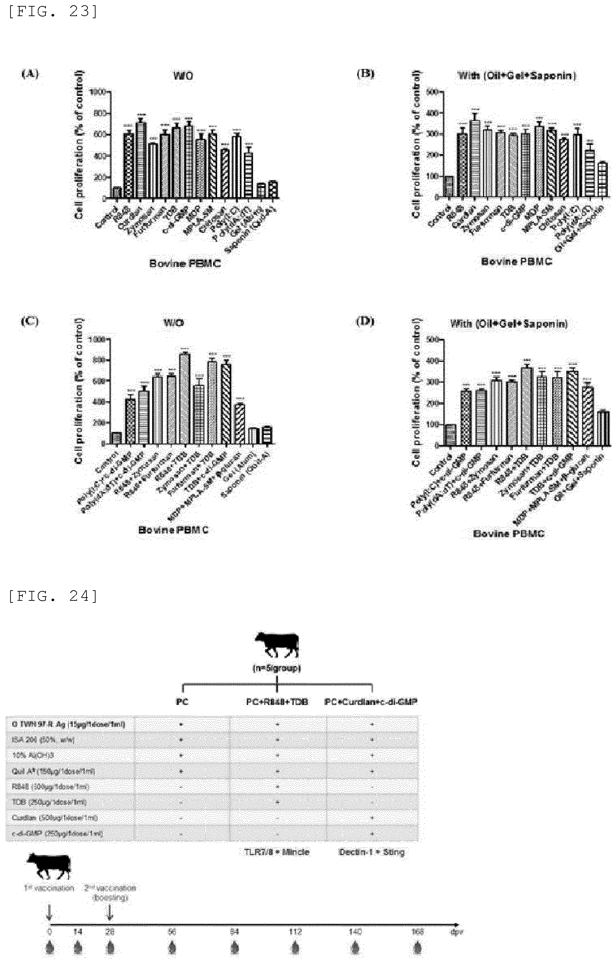

FIG. 23(A) shows the results of observing cell proliferation by BrdU incorporation 96 hours after treatment of the bovine-derived PBMCs with PRRs ligand and cytokine, oil, gel, saponin and Oil+Gel+Saponin, respectively. Cell proliferation was increased in all PRRs ligand-treated groups compared to the control group, and in particular, it was found that effects of Curdlan, TDB, c-di-GMP, R848 and Furfurman were great.

On the other hand, the effects of gel and saponin were insignificant at 96 hours. Further, after mixing each PRRs ligand and cytokine with Oil+Gel+Saponin and using the same for treatment, cell proliferation was observed. As a result of the experiment, the cell proliferation was increased in all PRRs ligands mixed with Oil+Gel+Saponin compared to the control group, but a level thereof was lower compared to the case where Oil+Gel+Saponin was not added (W/O) (FIG. 23(B)) .

FIG. 23(C) shows the result of observing cell proliferation by combining the PRRs ligands. The cell proliferation was increased in all treatments compared to the control group, and in particular, R848+TDB, Furfurman+TDB and TDB+c-di-GMP treatment groups showed higher values. From this result, it seems that the combination of these PRRs ligands effectively stimulates bovine PBMCs, thereby enhancing cell proliferation and stimulating immune response. Further, the immune response of bovine-derived PBMCs by these adjuvants is significantly higher than that of porcine-derived PBMCs, and therefore, it seems that bovine immune cells respond more sensitively than porcine immune cells.

Meanwhile, as a result of observing cell proliferation at 96 hours after treatment of the bovine-derived PBMCs with a mixture of the combination of PRRs ligands with Oil+Gel+Saponin, a level thereof was increased in the all PRRs ligands and Oil+Gel+Saponin mixture treatment groups compared to the control group. However, the above level was lower than no addition of Oil+Gel+Saponin (W/O) (FIG. 23(D)). From the above results, which are substantially similar to the results of porcine immune cells, it is considered that, when bovine immune cells are directly treated with PRRs ligand, cells are stimulated faster than when treated using the same along with Oil+Saponin+Gel, so as to promote the proliferation of cells and initiate an immune response.

For candidate substances showing significant results in PRRs ligand screening experiment using bovine PBMCs, animal experiments were performed with the strategy shown in FIG. 24 in order to observe the PRRs ligand-mediated adjuvant effects and memory response in actual field cattle. For the field experiment, 5 months old cattle (FMD antibody sero-negative) were used, and cattle were classified into 3 groups (n=5/group). Animals not vaccinated were used as a negative control group, and cattle were isolated in confined spaces during the experiment.

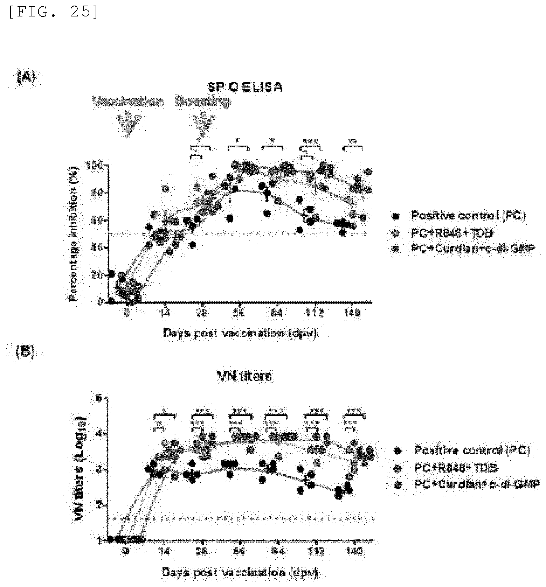

As a result of confirming Ab titers (antibody titer) by SP O ELISA, the PRRs ligand-added group showed significantly higher antibody titer at 28 days after vaccination (28 dpv) than the control group. Further, the high level was maintained till 140 days after boosting (140 dpv) (FIG. 25 (A)). Further, as a result of confirming VN titer (virus neutralization titer), the PRRs ligand-added group showed a significantly higher value than the 14 dpv control group (*p<0.01*)*,* and a very high level (*p<0.001*) was maintained from then to 140 dpv. (FIG. 25(B)).

From the above results, when adding R848+TDB, Curdlan+c-di-GMP, etc. as an adjuvant to a vaccine, innate and cellular immune responses are effectively stimulated, which in turn continuously stimulate humoral immune responses, thereby expecting to contribute to defense of host from FMDV infection.

### 2-2. Assessment of reactivity and safety of adjuvant candidates in pigs

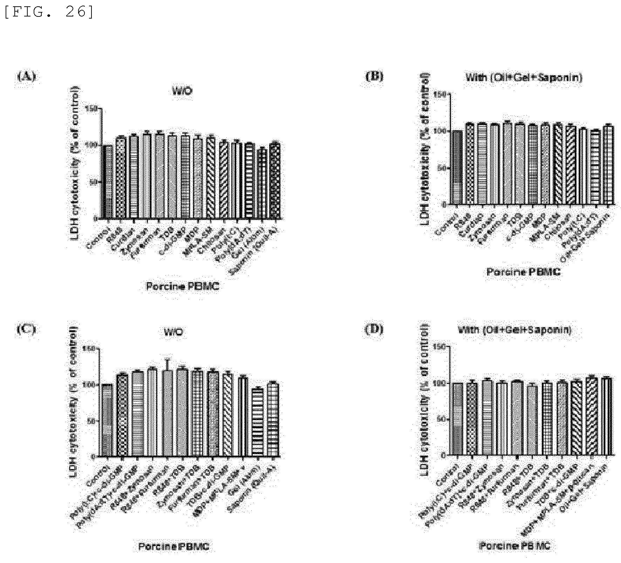

In order to observe cytotoxicity due to PRRs ligand and cytokine, oil, gel, saponin and Oil+Gel+Saponin, respectively, in porcine-derived PBMCs, LDH (Lactate Dehydrogenase) secretion was confirmed. As a result, it was found that the treatment group itself had low cytotoxicity, and there was no cytotoxicity at the adjuvant concentration used in this experiment (FIG. 26(A)).

FIG. 26(B) shows the results of observing cytotoxicity after treatment of porcine-derived PBMCs with a mixture of PRRs ligand and cytokines with Oil+Gel+Saponin as a vaccine adjuvant. Even at this time, cytotoxicity due to the adjuvant mixture was not observed as compared to the control group.

On the other hand, even when cytotoxicity was confirmed after treatment of porcine-derived PBMCs with combining PRRs ligand, cytotoxicity due to these adjuvants was also not observed (FIG. 26 (C)). Further, even after treatment of porcine-derived PBMCs with a mixture of the combined PRRs ligand and the vaccine adjuvant, that is, Oil+Gel+Saponin, a similar tendency was shown (FIG. 26(D)).

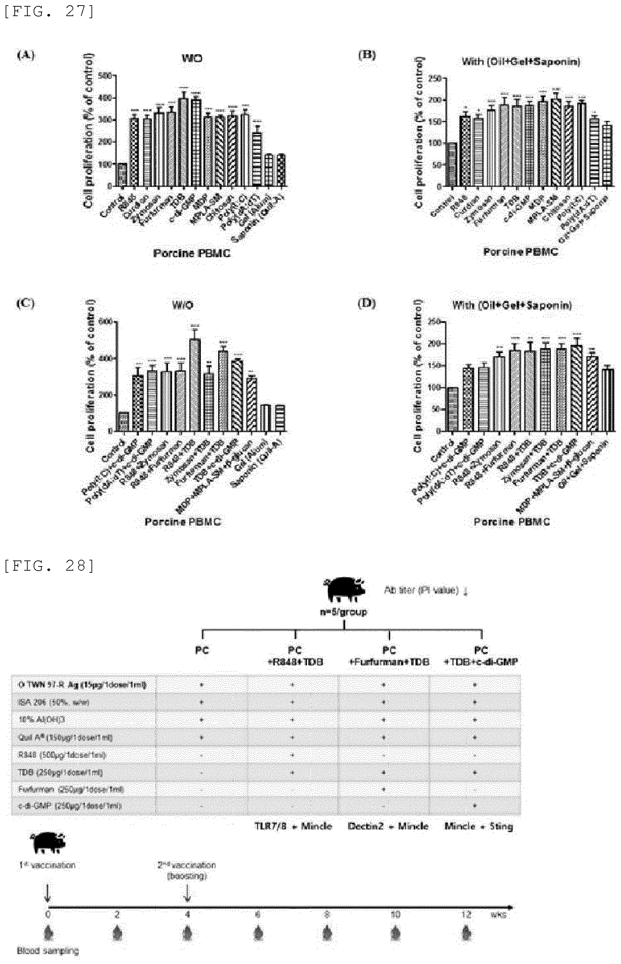

FIG. 27(A) shows that, as a result of observing cell proliferation by BrdU incorporation in porcine-derived PBMCs at 96 hours after treatment of the porcine-derive PBMCs with PRRs ligand and cytokine, oil, gel, saponoin and Oil+Gel+Saponin, respectively, the cell proliferation was increased in all PRRs ligand-treated groups as compared to the control group, and in particular, effects of TDB and c-di-GMP were found to be the greatest. On the other hand, effects of gel and saponin were insignificant at 96 hours.

As a result of observing cell proliferation at 96 hours after treatment of porcine-derived PBMCs with a mixture of PRRs ligand and cytokine with Oil+Gel+Saponin, the cell proliferation was increased in each of all PRRs ligand and Oil+Gel+Saponin mixture treatment groups as compared to the control group, however, a level thereof was lower than no addition of Oil+Gel+Saponin (W/O) (FIG. 27(B)). FIG. 27(C) shows the results of observing cell proliferation by combination of PRRs ligands. As compared to the control group, all treatment groups showed an increase in cell proliferation, in particular, R848+TDB, Furfurman+TDB and TDB+c-di-GMP treatment groups showed higher values. From these results, it is believed that a combination of these PRRs ligands effectively stimulates porcine PBMCs, thereby enhancing cell proliferation and stimulating immune responses. On the other hand, as a result of observing cell proliferation at 96 hours after treatment of the porcine-derived PBMCs with a mixture of the combined PRRs ligand with Oil+Gel+Saponin, a level thereof was increased in all PRRs ligand and Oil+Gel+Saponin mixture treatment groups as compared to the control group, but was lower than no addition of Oil+Gel+Saponin (W/O) (FIG. 27(D)). From the above results, it seems that, when the porcine immune cells are treated with PRRs ligand alone without any oil adjuvant (oil emulsion), the cells may be stimulated faster than when treated using the same along with Oil+Saponin+Gel, so as to promote diffusion of cells and initiate an immune response.

For candidate substances showing significant results in the PRRs ligand screening experiment using porcine PBMCs, animal experiments were performed as follows in order to observe PRRs ligand-mediated adjuvant effects and memory response in actual field pigs (FIG. 28). For the field experiment, 10-week-old pigs (FMD antibody sero-negative) were used and classified into 4 groups (n=5/group). Animals not vaccinated were used as a negative control group, and the pigs were isolated in confined spaces during the study.

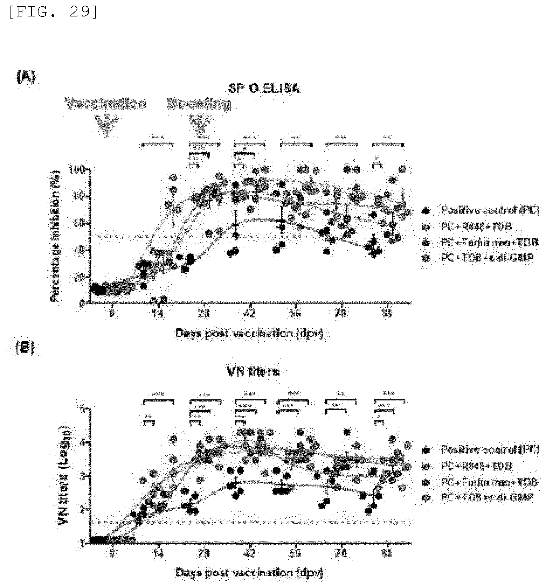

In order to confirm antibody titer by vaccination, SP O ELISA was performed using a porcine serum. As a result, as shown in FIG. 29(A), the antibody titer was significantly increased in the TDB+c-di-GMP administration group at 14 dpv (*p<0.001*)*,* as compared to the control group. Further, at 28 dpv, the antibody titer was significantly increased in each of all PRRs ligand administration groups (*p<0.001*) as compared to the control group. In particular, in the case of the TDB+c-di-GMP added group, it was found that the antibody titer at a significantly higher level than the control group was continuously maintained till 84 dpv. The high antibody titer by primary vaccination in pigs is interpreted as that PRRs ligands effectively activate innate and cellular immune responses and strongly induce humoral immune responses.

Further, it is believed that secondary vaccination (boosting) at 28 dpv may effectively induces 'recall stimulation' of the immune cells stimulated by the primary vaccination, while maintaining long-term immunity. Further, as a result of confirming the VN titer, TDB+c-di-GMP (*p<0.001*)*,* R848+TDB (*p<0.01*) added groups showed significantly higher VN titers than the control group at 14 dpv. Further, at 28 dpv, all PRRs added groups showed significantly higher VN titers than the control group (*p<0.001*)*.* In particular, the TDB+c-di-GMP added group maintained high VN titer till 84 dpv, and the R848+TDB added group showed excellent immunity enhancing effects from early stage (14 dpv) to mid-term (42 dpv). However, the above effects thereafter showed a tendency to be slightly decreased.

In addition, in the case of the Furfurman+TDB added group, the VN titer was increased somewhat slowly till the initial 14 dpv, but the VN titer was significantly higher than the control group from 28 dpv to 84 dpv.

The PRRs ligand+additive groups showed significantly higher levels from 14 dpv than the control group, and even till 84 dpv, a significantly higher level compared to the control group was maintained (FIG. 29(B)). From the above results, it is expected that, when R848+TDB, Furfurman+TDB and TDB+c-di-GMP are added as adjuvants to vaccines, the vaccine may effectively stimulate innate and cellular immune responses and continuously stimulate humoral immune responses, thereby contributing significantly to host defense from FMDV infection. Furthermore, it is expected that the present invention is possibly utilized in development of FMDV vaccine according to a new strategy.

## Claims

1. An adjuvant composition, comprising a pattern recognition receptors (PRRs) ligand or cytokine as an active ingredient.

2. An adjuvant composition, comprising a pattern recognition receptors (PRRs) ligand and cytokine as active ingredients.

3. The adjuvant composition according to claim 1 or 2, wherein the PRRs ligand includes any one or more among R848 (TLR-7/8 agonist), TDB (Mincle agonist), Curdlan (Dectin-1 agonist), Furfurman (Dectin-2 agonist), MDP (NOD-2 ligand), MPLA-SM (TLR-4), Zymosan (Dectin-2/TLR-2), Chitosan (NLRP inflammasome inducer, MR), c-di-GMP, c-di-AMP, cGAMP (STING ligand), Poly(I:C) (TLR-3) and Poly(dA:dT) (RIG-1/CDS agonist, AIM-2 inflammasome inducer).

4. The adjuvant composition according to claim 1 or 2, wherein the cytokine includes any one or more among IL-1 beta, IL-2, IL-4, IL-5, IL-6, IL-9, IL-10, IL-12p70, IL-12/23p40, IL-15/15R, IL-17A, IL-21, IL-22, IL-23, IL-33, TNF-alpha, and IFN-gamma.

5. The adjuvant composition according to claim 1 or 2, wherein the PRRs ligand is included in an amount of 1 to 100 µg/ml.

6. The adjuvant composition according to claim 3, wherein two PRRs ligands are included, and any one of the PRRs ligands : the remaining PRRs ligand are included in a ratio by weight of 1 : 1 to 3.

7. The adjuvant composition according to claim 6, wherein the PRRs ligand is included in an amount of 1 to 30 µg/ml.

8. The adjuvant composition according to claim 1 or 2 wherein the cytokine is included in an amount of 1 to 100 µg/ml.

9. The adjuvant composition according to claim 1 or 2, wherein the composition is used for a foot-and-mouth disease.

10. A vaccine composition comprising the adjuvant composition according to claim 1 or 2.
